# EUROPÄISCHE PATENTSCHRIFT

(11) **EP 3 106 107 B1**
(45) Veröffentlichungstag und Bekanntmachung des Hinweises auf die Patenterteilung: **28.11.2018**
(21) Anmeldenummer: 16171548.7
(22) Anmeldetag: 26.05.2016
(51) Int. Cl.: A61B 17/08

(54) **HERSTELLVERFAHREN FÜR EINE VORRICHTUNG ZUR ATRAUMATISCHEN VERSORGUNG VON WUNDEN**
PRODUCTION METHOD FOR A DEVICE FOR NON-TRAUMATIC CARING FOR WOUNDS
PROCÉDÉ DE FABRICATION POUR UN DISPOSITIF POUR LE SOIN ATRAUMATIQUE DE BLESSURES

(30) Priorität: 29.05.2015 DE 102015108503
(43) Veröffentlichungstag der Anmeldung: 21.12.2016
(73) Patentinhaber: Schnepel, Thede, 23701 Suesel (DE)
(72) Erfinder: Kaeßmann, Uwe, 24235 Brodersdorf (DE)
(74) Vertreter: Hauck Patentanwaltspartnerschaft mbB

(56) Entgegenhaltungen:
- WO-A1-2008/037280
- DE-B1- 2 038 038
- DE-B3-102007 001 278
- US-A1- 2013 014 355

## Beschreibung

Die vorliegende Erfindung betrifft ein Verfahren zur Herstellung einer erfindungsgemäßen Vorrichtung zur atraumatischen Versorgung von Wunden. Die Vorrichtung dient zur atraumatischen Versorgung von Wunden, insbesondere zum Wundverschluss und zur Wundabdeckung, mit einem Paar von Verschlussgliederreihen, die ineinandergreifend an einer Oberseite jeweils eines Tragbands befestigt sind und mit Hilfe eines Schiebers miteinander verbindbar und voneinander lösbar sind, mindestens einem Abstandspolster, das eine Auflageseite zur Auflage auf der Wunde und eine Oberseite aufweist, und das mit seiner Oberseite an der den Verschlussgliederreihen gegenüberliegenden Unterseite des jeweils einen Tragbands befestigt ist.

Darüber hinaus betrifft die Erfindung eine Vorrichtung zur Herstellung der Vorrichtung zur atraumatischen Versorgung von Wunden.

Aus dem Stand der Technik sind verschiedene Arten von Wundreißverschlüssen bekannt, welche dem Wundverschluss und der Wundabdeckung von beispielsweise Operations- oder Gelegenheitswunden dienen und zwei ineinandergreifende und jeweils an einem Tragband befestigte Verschlussgliederreihen, einen Schieber und ein an den Tragbändern befestigtes Abstandspolster aufweisen.

DE 44 00 732 A1 beschreibt einen Wundreißverschluss mit großflächig ausgebildeten Tragbändern. Die Tragbänder bestehen aus einem festen Material und sind in ihrer Längsrichtung entlang einer Faltkante zu einem Doppeltragbandbereich gefaltet. An den Tragbändern ist ein Abstandspolster vorgesehen, das einen Wundfreiraum bildet. Nachteilig an der beschriebenen Konstruktion ist, dass aus der Wunde abgegebene Körperflüssigkeit mit den Verschlussgliederreihen in Berührung kommen und diese verkleben können. Hierdurch kann eine Blockade des Wundreißverschlusses auftreten.

Eine weitere Konstruktion eines Wundreißverschlusses ist in DE 37 06 599 C2 sowie EP 0 271 741 B1 beschrieben. Der bekannte Wundreißverschluss besteht ebenfalls aus Tragbändern und daran befestigten Verschlussreihen, die über Abstandspolster von der Wunde beabstandet sind. Auch bei diesem Wundreißverschluss kann ausgetretene Wundflüssigkeit in den Reißverschluss gelangen und diesen blockieren.

Aus US 3,863,640 ist ein Wundverschluss bekannt, der einen wiederverschließbaren Gleitverschluss besitzt. Die Unterseite des Gleitverschlusses ist mit einer Klebeschicht versehen. Ferner ist bekannt, im zentralen Bereich des Wundverschlusses eine Gazeschicht oder eine andere absorbierende Wundauflage vorzusehen. Auch bei diesem Wundverschluss besteht die Gefahr, dass austretende Wundflüssigkeit den wiederverschließbaren Gleitverschluss zusetzt und verklebt. Aus DE 61 83 93 ist ein lösbarer Wundverband bekannt, der mit einem Reißverschluss geöffnet werden kann.

Aus US 2,012,755 ist ein Wundverband mit einem Reißverschluss bekannt, bei dem ein selbstklebendes Pflaster mit einem elastischen Streifen vernäht ist, auf dem ein Reißverschluss gehalten ist. Die Verschlussgliederreihen des Reißverschlusses sind im Wundbereich mit einem vorstehenden elastischen Streifen abgedeckt.

Aus DE 34 44 782 ist ein temporärer Wundverschluss mit einem Gleitschieber bekannt. Der Gleitschieber ist zur Wunde hin mit einem Untertrittstreifen abgedeckt. Aus DE 10 2004 059 499 A1 ist ein Wundreißverschluss mit einem semiresorptiven Abstandspolster bekannt. Die Abstandspolster weisen eine Trennschicht auf, die einen Flüssigkeitsdurchtritt durch eine Resorptionsschicht des Abstandspolsters verhindert.

Die EP 1 941 837 B1 sowie die DE 10 2007 001 278 B3 offenbaren eine Vorrichtung zur atraumatischen Versorgung von Wunden, bei der austretende Wundflüssigkeiten durch Verwendung von sich überlappenden Folienbändern aufgehalten wird. Diese Folienbänder sind so ausgebildet, dass das eine Folienband weiter über das Abstandspolster vorsteht als das gegenüberliegende Folienband und dass, bei geschlossenen Verschlussgliederreihen, die Folienbänder so ineinandergeschoben sind, dass das weiter vorstehende Folienband zwischen eine der Verschlussgliederreihen und das gegenüberliegende Folienband geschoben ist. Die Folienbänder sollen die Wunde von den Verschlussgliederreihen abschirmen. Nachteilig ist hier allerdings zum einen der hohe Produktionsaufwand, bedingt durch das Aufbringen der Folienbänder.

WO 2008/037280 A1 beschreibt einen Wundverschluss mit einem Reißverschluss, der zwei Tragbänder mit daran angeordneten Verschlussgliederreihen aufweist. Jedes Tragband weist auf der der Wunde zugekehrten Unterseite mit Abstand von den Verschlussgliederreihen einen freiraumbildenden Distanzstreifen auf. Eine Tragbandschicht der Tragbänder erstreckt sich dabei über die gesamte Breite des jeweiligen Tragbandes über die Breite des jeweiligen Distanzstreifens hinaus. Hierbei wird ein streifenförmiger Überstand gebildet, der eine Klebstoffbeschichtung zum Verkleben mit der Haut des Patienten aufweist.

US 2013/0014355 A1 betrifft einen wasserdichten Reißverschluss mit einem wasserdichten Film aus Polyurethan, dessen eine Seite bedruckt ist. Nach Aufbringen der wasserdichten Schicht wird diese der Länge nach aufgeschnitten.

DE 20 38 038 B1 beschreibt einen Reißverschluss mit zwei Tragbändern, wobei die Tragbänder auf einer Seite mit einer die Tragbänder über eine Verschlusstrennlinie hinweg verbindenden Deckschicht versehen sind.

Es ist die technische Aufgabe der vorliegenden Erfindung, ein Verfahren und eine Vorrichtung zur Herstellung eines Wundverschlusses zur atraumatischen Versorgung von Wunden bereitzustellen, welche in einfacher und zuverlässiger Weise einen vorteilhaften Wundverschluss schaffen.

Die Erfindung löst die Aufgabe durch die Merkmale der unabhängigen Ansprüche 1 und 7. Vorteilhafte Ausgestaltungen sind Gegenstand der Unteransprüche.

Die Erfindung betrifft ein Verfahren zur Herstellung einer erfindungsgemäßen Vorrichtung zur atraumatischen Versorgung von Wunden. Das Verfahren ist dadurch gekennzeichnet, dass ein durchgehendes Tragband an der den Verschlussgliederreihen gegenüberliegenden Unterseite mit der Oberseite mindestens eines durchgehenden Abstandspolster verbunden wird und anschließend das mindestens eine Abstandspolster einschließlich des damit verbundenen Tragbandes aus Richtung der Auflageseite des Abstandspolsters entlang einer Linie zwischen den Verschlussgliederreihen durchtrennt wird. Die Verschlussgliederreihen sind also anfangs im gekuppelten Zustand auf einem geschlossenen Tragband aufgebracht. Nach dem Durchtrennen besteht das mindestens eine Abstandspolster aus zwei Teilen, welche an jeweils einem, nun ebenfalls zweiteiligen, Tragband befestigt sind. Das Durchtrennen erfolgt von unten, also von der späteren Wundauflageseite der erfindungsgemäßen Vorrichtung, derart, dass die während dieses Verfahrens geschlossenen Verschlussgliederreihen nicht beschädigt werden. Durch dieses Herstellungsverfahren wird ein besonders bündiger Abschluss der beiden Tragbänder miteinander erreicht und sichergestellt, dass die Verschlussgliederreihen so ineinandergreifen, dass die Tragbänder im gekuppelten Zustand der Verschlussgliederreihen im Wesentlichen flüssigkeitsundurchlässig abschließen. Weiterhin ermöglicht dieses Herstellungsverfahren die Verwendung eines Wundpflasters mit einer Wundauflage auf einem Trägermaterial, wobei das mindestens eine Abstandspolster durch die Wundauflage gebildet ist. Optional weist das Trägermaterial auf seiner zur Wundauflage zugewandten Seite ein Paar von selbstklebenden Streifen auf. Insbesondere kann das Abstandspolster Teil eines handelsüblichen Wundheilpflasters sein.

In einer bevorzugten Ausgestaltung wird vor dem Verbinden des durchgehenden Tragbandes mit dem durchgehenden Abstandspolster auf der Unterseite des durchgehenden Tragbands eine flüssigkeitsundurchlässige Beschichtung, insbesondere bestehend aus Polyurethan, aufgebracht. Hierzu kann beispielsweise eine fertige und ausgehärtete Beschichtung mit dem Tragband verbunden werden oder eine noch nicht ausgehärtete Schicht aufgebracht werden.

Das mindestens eine Abstandspolster kann bevorzugt durch Ultraschallschweißen an dem durchgehenden Tragband befestigt sein. Möglich ist aber beispielsweise auch ein Vernähen oder Kleben.

In einer bevorzugten Ausgestaltung erfolgt das Durchtrennen des mindestens einen Abstandspolsters einschließlich des damit verbundenen Tragbandes durch Laserschneiden. Dies erlaubt ein exaktes Schneiden ohne die Verschlussgliederreihen zu beschädigen. Es kann aber auch vorgesehen sein, die Verschlussgliederreihen während des Schneidens zu schützen, beispielsweise durch eine Abschirmung.

In einer Ausgestaltung können auf die Oberseite der Tragbänder, auf beiden Seiten des Paares von Verschlussgliederreihen, Dichtauflagen, insbesondere bestehend aus thermoplastischen Schmelzfolien, aufgebracht werden. Diese Dichtauflagen können vor oder nach dem Verbinden des durchgehenden Tragbands mit dem mindestens einen durchgehenden Abstandspolster oder auch nach dem Durchtrennen des mindestens einen Abstandspolsters einschließlich des damit verbundenen Tragbandes aufgebracht werden. Die Dichtauflagen können beispielsweise durch Kleben oder durch Applikation von Wärme befestigt werden.

In einer weiteren Ausgestaltung können auf der Oberseite der Tragbänder, auf beiden Seiten des Paares von Verschlussgliederreihen, Abdeckungen, insbesondere bestehend aus Polyester-Vliesen, aufgebracht werden, welche über die Tragbänder überstehen. Bevorzugt werden diese Abdeckungen nach dem Verbinden oder während des Verbindens des durchgehenden Tragbands mit dem mindestens einen durchgehenden Abstandspolster oder auch vor oder nach dem Durchtrennen des mindestens einen Abstandspolsters einschließlich des damit verbundenen Tragbandes aufgebracht. Die Abdeckungen können mit den Tragbändern beispielsweise verklebt werden. Bevorzugt werden die Abdeckungen gemeinsam mit dem durchgehenden Tragband mit dem mindestens einen durchgehenden Abstandspolster verbunden. Die über die Tragbänder überstehenden Abdeckungen können weiterhin auch mit dem Trägermaterial eines bevorzugten Wundpflasters beispielsweise durch Kleben verbunden werden.

Die Offenbarung betrifft weiterhin eine Vorrichtung zur Herstellung einer Vorrichtung zur atraumatischen Versorgung von Wunden. Diese Herstellungsvorrichtung weist eine Verbindeeinheit auf, welche dazu ausgestaltet ist, die den Verschlussgliederreihen gegenüberliegende Unterseite des durchgehenden Tragbands mit der Oberseite des mindestens einen durchgehenden Abstandspolsters zu einer Einheit zu verbinden. Darüber hinaus weist die Herstellungsvorrichtung eine Schneideeinheit auf, welche dazu ausgestaltet ist, die Einheit aus Tragband und Abstandspolster aus Richtung der Auflageseite des Abstandspolsters entlang einer Linie zwischen den Verschlussgliederreihen derart zu durchtrennen, dass die Verschlussgliederreihen nach dem Durchtrennen an jeweils einem Tragband und Abstandspolster befindlich sind. Das Durchtrennen von Tragband und Abstandspolster erfolgt bevorzugt mittig zwischen den Verschlussgliederreihen.

In einer bevorzugten Ausgestaltung weist die Verbindeeinheit eine Ultraschallschweißeinheit zur Verbindung des Tragbands mit dem mindestens einen Abstandspolster auf.

In einer bevorzugten Ausgestaltung weist die Schneideeinheit eine Lasereinheit zum Durchtrennen der Einheit aus Tragband und Abstandspolster auf.

Die hergestellte Vorrichtung dient der atraumatischen Versorgung von Wunden, insbesondere zum Wundverschluss und zur Wundabdeckung. Die Vorrichtung besteht aus einem Paar von Verschlussgliederreihen, die ineinandergreifend an einer Oberseite jeweils eines Tragbands befestigt sind und mit Hilfe eines Schiebers miteinander verbindbar und voneinander lösbar sind, mindestens einem Abstandspolster, das eine Auflageseite zur Auflage auf der Wunde und eine Oberseite aufweist, und das mit seiner Oberseite an der den Verschlussgliederreihen gegenüberliegenden Unterseite des jeweils einen Tragbands befestigt ist. Die Tragbänder weisen auf ihrer Unterseite jeweils eine flüssigkeitsundurchlässige Beschichtung auf. Weiterhin greifen die Verschlussgliederreihen so ineinander, dass die Tragbänder im gekuppelten Zustand der Verschlussgliederreihen flüssigkeitsundurchlässig abschließen. Flüssigkeitsundurchlässig bedeutet, dass nicht unter Druck stehende, wässrige Flüssigkeiten, wie beispielsweise aus der Wunde austretendes Blut oder auch Sekret die Beschichtung nicht durchdringen können.

Die Verschlussgliederreihen können beispielsweise durch einen Reißverschluss oder aber auch durch einen Gleitverschluss oder einen sonstigen wiederverschließbaren Verschluss gebildet sein. Durch einen Schieber können die beiden Verschlussgliederreihen miteinander gekuppelt und bei Bedarf wieder entkuppelt werden, zum Beispiel um die Wundheilung zu inspizieren. Die Verschlussgliederreihen sind auf den Oberseiten jeweils eines Tragbands aufgebracht. Die Vorrichtung besitzt ferner ein oder mehrere Abstandspolster, die an den Unterseiten jeweils eines Tragbands angeordnet sind. Dieses mindestens eine Abstandspolster dient zum einen als Wundauflage, also zum Schutz der Wunde sowie zur Aufnahme aus der Wunde austretender Flüssigkeit und Sekret, sowie zum Anderen als Abstandsgeber zwischen der Wunde und den Verschlussgliederreihen.

Die Tragbänder weisen auf ihrer Unterseite eine flüssigkeitsundurchlässige Beschichtung auf. Diese Beschichtung verhindert ein Durchtreten von Wundflüssigkeiten durch das Abstandspolster in die darüber liegenden Tragbänder. Weiterhin greifen die Verschlussgliederreihen so ineinander, dass die Tragbänder im gekuppelten Zustand der Verschlussgliederreihen flüssigkeitsundurchlässig abschließen. Ein solcher flüssigkeitsundurchlässiger Abschluss kann dadurch erreicht werden, dass die Tragbänder im geschlossenen Zustand der Verschlussgliederreihen bündig aneinanderstoßen und verhindert einen Austritt von Wundflüssigkeit in die Kuppelglieder. Durch die Kombination dieser beiden Merkmale ist eine flüssigkeitsundurchlässige Abschirmung der Wunde gegenüber den Verschlussgliederreihen auch ohne die Verwendung von überlappenden Folienbändern gegeben. Durch eine möglichst großflächige flüssigkeitsundurchlässige Beschichtung der Unterseite der Tragbänder kann eine Wunde insbesondere auch gegen Flüssigkeitseintritt von außen, wie beispielsweise durch Spritzwasser, geschützt werden. Auch wird so die Aufnahmekapazität des Abstandspolsters für Flüssigkeiten nicht durch Flüssigkeiten von außen negativ beeinträchtigt. Die Vorrichtung verhindert auf eine einfache und zuverlässige Weise ein Verkleben der Verschlussgliederreihen durch Blut oder andere Wundflüssigkeiten bei gleichzeitigem Schutz der Wunde. Der Verzicht auf Folienbänder erhöht die Wirkweise des Pflasters, da Wundflüssigkeiten noch zuverlässiger von den Verschlussgliederreihen ferngehalten werden, und erleichtert darüber hinaus die Fertigung der erfindungsgemäßen Vorrichtung.

Das mindestens eine Abstandspolster kann beispielsweise durch eine Naht, durch Kleben und insbesondere durch Ultraschallschweißen an dem jeweiligen Tragband befestigt sein. Die Befestigung kann entlang dem Umfang oder flächig erfolgen.

In einer Ausgestaltung ist mindestens eine der flüssigkeitsundurchlässigen Beschichtungen der Tragbänder aus Polyurethan. Polyurethan eignet sich besonders aufgrund der einfachen Verarbeitung sowie aufgrund seiner Hautverträglichkeit.

In einer weiteren Ausgestaltung bildet das mindestens eine Abstandspolster im gekuppelten Zustand der Verschlussgliederreihen eine ebene Auflageseite, so dass die Tragbänder nicht mit der Wunde in Berührung kommen, sondern lediglich das Abstandspolster auf der Wunde aufliegt. Der Abstand ist dabei bevorzugt derart bemessen, dass auch beim Öffnen um Schließen der Verschlussgliederreihen kein Kontakt mit der Wunde entsteht.

In einer Ausgestaltung ist ein Wundpflaster mit einer Wundauflage auf einem Trägermaterial vorgesehen, wobei das mindestens eine Abstandspolster durch die Wundauflage gebildet ist. Insbesondere kann das Trägermaterial auf seiner zur Wundauflage zugewandten Seite ein Paar von selbstklebenden Streifen aufweisen. Die erfindungsgemäße Vorrichtung kann über die selbstklebenden Streifen mit der die Wunde umgebenden Haut verbunden werden. Die Streifen können beispielsweise durch einen Acrylkleber selbstklebend sein.

In einer weiteren Ausgestaltung können auf der Oberseite der Tragbänder, auf beiden Seiten des Paares von Verschlussgliederreihen, Dichtauflagen aufgebracht sein. Diese Dichtauflagen können die Wunde und das Abstandspolster noch weiter vor einem Flüssigkeitseintritt von außen schützen. Insbesondere können diese Dichtauflagen thermoplastische Schmelzfolien sein.

In einer weiteren Ausgestaltung sind auf der Oberseite der Tragbänder, auf beiden Seiten des Paares von Verschlussgliederreihen, Abdeckungen aufgebracht, welche über die Tragbänder überstehen. Diese Abdeckungen können Polyestervliese sein, insbesondere können sie aus dem gleichen Material gefertigt sein wie das Trägermaterial des Wundpflasters. Diese Abdeckung kann der Abdeckung der Kanten der Oberseiten der Tragbänder dienen und vor allem optische Vorzüge haben. Die über die Tragbänder überstehenden Abdeckungen können weiterhin auch mit dem Trägermaterial eines bevorzugten Wundpflasters beispielsweise durch Kleben verbunden sein.

Ein Ausführungsbeispiel der Erfindung wird nachfolgend anhand einer Figur näher erläutert. Es zeigt:

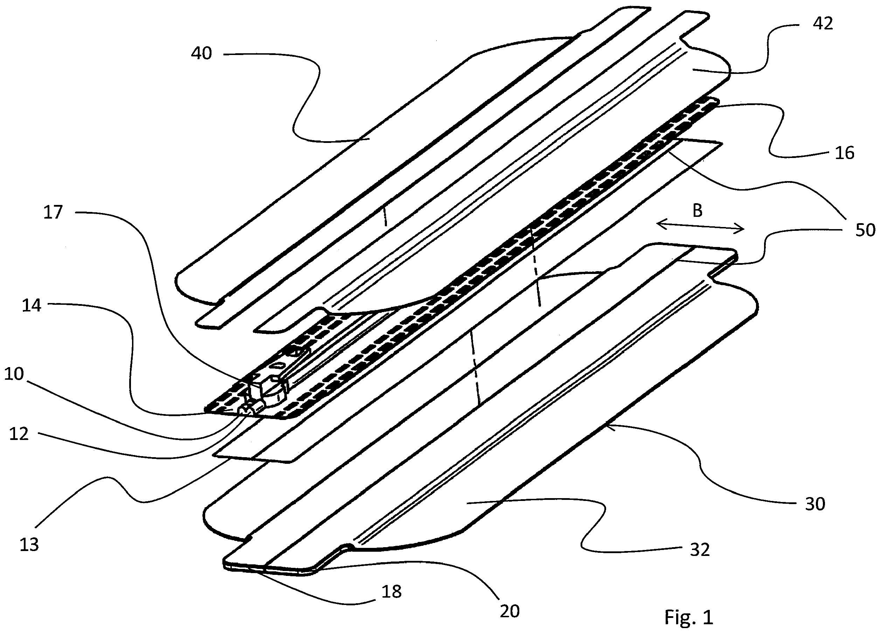

- Fig. 1: eine Vorrichtung zur atraumatischen Versorgung von Wunden in einer Explosionsdarstellung.

Figur 1 zeigt eine erfindungsgemäße Vorrichtung zur atraumatischen Versorgung von Wunden in einer Explosionsdarstellung, bestehend aus einem Paar Verschlussgliederreihen 10, 12 befestigt auf jeweils einem Tragband 14, 16. Die Verschlussgliederreihen 10, 12 sind mit Hilfe eines Schiebers 17 miteinander verbindbar und voneinander lösbar ausgebildet. Wie der Figur 1 zu entnehmen, liegen die Verschlussgliederreihen 10, 12 auf einer Oberseite der Tragbänder 14, 16 auf, so dass im gekuppelten Zustand der Verschlussgliederreihen 10, 12 die Tragbänder 14, 16 bündig und flüssigkeitsundurchlässig aneinandergrenzen. Weiterhin weisen die Tragbänder 14, 16 auf einer wundseitigen Unterseite eine flüssigkeitsabweisende Beschichtung 13 auf. Diese Beschichtung 13 kann aus Polyurethan sein und beispielsweise durch Streichen auf die Unterseite der Tragbänder 14, 16 aufgebracht sein.

Unter den mit der flüssigkeitsundurchlässigen Beschichtung 13 versehenen Verschlussgliederreihen 10, 12 ist jeweils ein Abstandspolster 18, 20 vorgesehen.

Die Abstandspolster 18, 20 dienen der Beabstandung von Wunde (nicht gezeigt) und Tragbändern 14, 16 sowie der Wundauflage zur Aufnahme von Wundflüssigkeit. Die Tragbänder 14, 16 sind zusammen mit den darauf befindlichen Verschlussgliederreihen 10, 12 mittels Ultraschallschweißen auf den Abstandspolstern 18, 20 befestigt. Alternativ ist aber auch ein Verkleben oder ein Vernähen möglich. In diesem Ausführungsbeispiel reichen die Tragbänder 14, 16 über die ganze Breite B der Abstandpolster 18, 20. Die Tragbänder 14, 16 können jedoch auch eine abweichende Breite aufweisen.

Die Abstandspolster 18, 20 sind durch die Wundauflage eines Wundpflasters 30 gebildet. Das Wundpflaster 30 besteht aus einem Abstandspolster 18, 20 aus beispielsweise Viskose-Vlies, einem Trägermaterial 32 aus beispielsweise Polyester-Vlies und einer Schutzfolie (nicht gezeigt) an der selbstklebenden Unterseite des Trägermaterials 32. Die klebende Unterseite des Trägermaterials 32 kann beispielsweise ein Polyacrylatkleber sein und dient dem Aufkleben der Vorrichtung auf die Haut. Das Abstandspolster 18, 20 ist an dem Trägermaterial 32 befestigt. Es kann sich insbesondere um ein handelsübliches Wundpflaster handeln, das beispielsweise als Endlosware produziert und auf die gewünschte Länge zurechtgeschnitten werden kann.

Weiterhin zeigt Figur 1 Abdeckungen 40, 42, welche auf der Oberseite der Tragbänder 14, 16 auf beiden Seiten des Paares von Verschlussgliederreihen 10, 12 aufgebracht sind. Die Abdeckungen 40, 42 können beispielsweise Polyester-Vliese sein und ragen über die Kanten der Tragbänder 14, 16 hinaus. In der dargestellten Ausführung stehen die Abdeckungen 40, 42 gerade soweit über die Kanten der Tragbänder 14, 16 über, dass sie das Trägermaterial 32 vollständig abdecken.

Das Herstellungsverfahren sieht vor, dass die Tragbänder 14, 16 zu Beginn ein durchgehendes Tragband bilden, welches auf der Oberseite die Verschlussgliederreihen 10, 12 aufweist und dessen Unterseite mit einer flüssigkeitsundurchlässigen Beschichtung 13 versehen ist. Erfindungsgemäß werden die Tragbänder 14, 16 mit Ultraschall mit den Abstandspolstern 18, 20, welche zu diesem Zeitpunkt ebenfalls als ein durchgehendes Abstandspolster ausgebildet sind, verschweißt. Im zweiten erfindungsgemäßen Verfahrensschritt werden schließlich das durchgehende Abstandspolster wie auch das darauf befindliche durchgehende Tragband einschließlich der flüssigkeitsundurchlässigen Beschichtung 13 mit einem Laser von der Wundseite her durchtrennt. Dadurch entsteht ein Spalt 50, welcher das durchgehende Abstandspolster in die beiden Abstandspolster 18, 20 und das durchgehende Tragband in die beiden Tragbänder 14, 16 teilt.

### Bezugszeichenliste

- 10: Verschlussgliederreihe
- 12: Verschlussgliederreihe
- 13: Flüssigkeitsundurchlässige Beschichtung
- 14: Tragbänder
- 16: Tragbänder
- 17: Schieber
- 18: Abstandspolster
- 20: Abstandspolster
- 30: Wundpflaster
- 32: Trägermaterial
- 40': Abdeckung
- 42: Abdeckung
- 50: Spalt

## Patentansprüche

1. Verfahren zur Herstellung einer Vorrichtung zum Wundverschluss und zur Wundabdeckung mit
- einem Paar von Verschlussgliederreihen (10, 12), die ineinandergreifend auf einer Oberseite eines durchgehenden, sich quer zum Verlauf der Verschlussgliederreihen erstreckenden, Tragbands (14, 16) befestigt sind und mit Hilfe eines Schiebers (17) miteinander verbindbar und voneinander lösbar sind,
- mindestens einem durchgehenden, sich quer zum Verlauf der Verschlussgliederreihen erstreckenden, Abstandspolster (18, 20) als Wundauflage, das eine Auflageseite zur Auflage auf der Wunde und eine Oberseite aufweist,
wobei die Arbeitschritte des Verfahrens beinhalten:
- das Verbinden der den Verschlussgliederreihen (10, 12) gegenüberliegenden Unterseite des durchgehenden Tragbands (14, 16) mit der Oberseite des mindestens einen durchgehenden Abstandspolsters (18, 20),
- das Durchtrennen des mindestens einen durchgehenden Abstandspolsters (18, 20) einschließlich des damit verbundenen, durchgehenden Tragbandes (14, 16) aus Richtung der Auflageseite des Abstandspolsters (18, 20) entlang einer Linie zwischen den Verschlussgliederreihen (10, 12) derart, dass nach dem Durchtrennen jede der Verschlussgliederreihen (10, 12) an einem Tragband (14, 16) und einem Abstandspolster (18, 20) befestigt ist.

2. Verfahren nach Anspruch 1, **dadurch gekennzeichnet, dass** vor dem Verbinden auf der Unterseite des durchgehenden Tragbands (14, 16) eine flüssigkeitsundurchlässige Beschichtung (13), bestehend aus Polyurethan, aufgebracht wird.

3. Verfahren nach Anspruch 1 oder 2, **dadurch gekennzeichnet, dass** das durchgehende Tragband (14, 16) mit dem durchgehenden Abstandspolster (18, 20) durch Ultraschallschweißen verbunden wird.

4. Verfahren nach einem der Ansprüche 1 bis 3, **dadurch gekennzeichnet, dass** das durchgehenden Abstandspolster (18, 20) einschließlich des damit verbundenen durchgehenden Tragbandes (14, 16) durch Laserschneiden durchtrennt wird.

5. Verfahren nach einem der Ansprüche 1 bis 4, **dadurch gekennzeichnet, dass** auf der Oberseite der Tragbänder (14, 16), auf beiden Seiten des Paares von Verschlussgliederreihen (10, 12), Dichtauflagen, bestehend aus thermoplastischen Schmelzfolien, aufgebracht werden.

6. Verfahren nach einem der Ansprüche 1 bis 5, **dadurch gekennzeichnet, dass** auf der Oberseite der Tragbänder (14, 16), auf beiden Seiten des Paares von Verschlussgliederreihen (10, 12), Abdeckungen (40,42), bestehend aus Polyester-Vliesen, aufgebracht werden, welche über die Tragbänder (14, 16) überstehen.

## Claims

1. A method for producing a device for wound closure and wound coverage having:
- a pair of mating rows of locking links (10, 12) that are fastened to a top side of a continuous support strip (14, 16) extending transversely to the path of the rows of locking links and are connectable with each other and releasable from each other with the assistance of a slider (17),
- at least one continuous spacing pad (18, 20) extending transversely to the path of the rows of locking links as a wound dressing which has a dressing side for placement on the wound and a top side,
wherein the steps of the method include:
- connecting the bottom side of the continuous support strip (14, 16) opposite the rows of locking links (10, 12) to the top side of the at least one continuous spacing pad (18, 20),
- severing the at least one continuous spacing pad (18, 20), including the continuous support strip (14, 16) connected thereto from the direction of the placement side of the spacing pad (18, 20) along a line between the rows of locking links (10, 12) such that after severing, each of the rows of locking links (10, 12) is fastened to a support strip (14, 16), and a spacing pad (18, 20).

2. The method according to claim 1, **characterized in that** a liquid-impermeable coating (13) consisting of polyurethane is applied to the bottom side of the continuous support strip (14, 16) before connecting to the bottom side.

3. The method according to claim 1 or 2, **characterized in that** the continuous support strip (14, 16) is connected to the continuous spacing pad (18, 20) by ultrasonic welding.

4. The method according to one of claims 1 to 3, **characterized in that** the continuous spacing pad (18, 20) including the continuous support strip (14, 16) connected thereto is severed by laser cutting.

5. The method according to one of claims 1 to 4, **characterized in that** sealing overlays consisting of thermoplastic fusible films are applied to the top side of the support strips (14, 16) on both sides of the pair of rows of locking links (10, 12).

6. The method according to one of claims 1 to 5, **characterized in that** sealing covers (40, 42) consisting of polyester fleeces which project above the support strips (14, 16) are applied to the top side of the support strips (14, 16) on both sides of the pair of rows of locking links (10, 12).

## Revendications

1. Procédé de fabrication d'un dispositif pour la fermeture des plaies et le pansement pour plaies, comprenant :
- une paire de rangées d'éléments de fermeture (10, 12) fixés de façon entrelacée sur un côté supérieur d'une bande de support continue (14, 16) s'étendant transversalement au parcours des rangées d'éléments de fermeture et aptes à être reliés les uns aux autres et détachés les uns des autres à l'aide d'un poussoir (17),
- au moins un coussinet d'espacement continu (18, 20) s'étendant transversalement au parcours des rangées d'éléments de fermeture, en tant que couverture de plaie, présentant une face de couverture pour couvrir la plaie et une face supérieure,
dans lequel les étapes d'exécution du procédé comprennent :
- l'assemblage de la face inférieure de la bande de support continue (14, 16) opposée aux rangées d'éléments de fermeture (10, 12) avec la face supérieure de l'au moins un coussinet d'espacement continu (18, 20),
- le sectionnement de l'au moins un coussinet d'espacement continu (18, 20), y compris la bande de support continue (14, 16) reliée à celui-ci, dans la direction de la face de couverture du coussinet d'espacement (18, 20), le long d'une ligne entre les rangées d'éléments de fermeture (10, 12), de telle façon qu'après le sectionnement, chacune des rangées d'éléments de fermeture est fixée à une bande de support (14, 16) et à un coussinet d'espacement (18, 20).

2. Procédé selon la revendication 1, **caractérisé en ce qu'**avant l'assemblage sur la face inférieure de la bande de support continue (14, 16), un revêtement imperméable au liquide (13) constitué de polyuréthane est appliqué.

3. Procédé selon la revendication 1 ou 2, **caractérisé en ce que** la bande de support continue (14, 16) est reliée au coussinet d'espacement continu (18, 20) par soudage aux ultrasons.

4. Procédé selon l'une des revendications 1 à 3, **caractérisé en ce que** le coussinet d'espacement continu (18, 20), y compris la bande de support continue (14, 16) reliée à celui-ci, est sectionné par découpe au laser.

5. Procédé selon l'une des revendications 1 à 4, **caractérisé en ce que** des couches d'étanchéité constituées de feuilles fusibles sont appliquées sur la face supérieure des bandes de support (14, 16), des deux côtés de la paire de rangées d'éléments de fermeture (10, 12).

6. Procédé selon l'une des revendications 1 à 5, **caractérisé en ce que** des recouvrements (40, 42) constitués d'un non-tissé de polyester sont appliqués sur la face supérieure des bandes de support (14, 16), des deux côtés de la paire de rangées d'éléments de fermeture (10, 12), en faisant saillie au-delà des bandes de support (14, 16).
